# FASCICULE DE BREVET EUROPEEN

(11) **EP 0 955 915 B1**
(45) Date de publication et mention de la délivrance du brevet: **09.04.2003**
(21) Numéro de dépôt: 95933449.1
(22) Date de dépôt: 28.09.1995
(51) Int. Cl.: B23K 26/00, H01S 3/16, A61B 17/22, A61C 1/00

(54) **APPAREIL LASER A EMISSION PULSEE, UTILISE DANS LE DOMAINE MEDICAL**
LASERVORRICHTUNG MIT PULSIERENDER EMISSION FÜR DIE MEDIZINTECHNIK
PULSED-EMISSION LASER APPARATUS FOR MEDICAL USE

(30) Priorité: 29.09.1994 FR 9411865
(43) Date de publication de la demande: 17.11.1999
(73) Titulaire: LOKKI S.A., 38200 Vienne (FR)
(72) Inventeur: GUILLET, Hubert, Gaston, F-38200 Vienne (FR)
(74) Mandataire: Thibault, Jean-Marc
(86) Numéro de dépôt international: FR9501258
(87) Numéro de publication internationale: WO96009799

(56) Documents cités:
- EP-A- 0 226 790
- EP-A- 0 443 751
- WO-A-92/07522
- FR-A- 2 607 329
- US-A- 4 039 970
- OPTICS LETTERS, vol. 14, no. 13, 1 Juillet 1989 pages 674-676, XP 000068931 HANSON F 'LASER-DIODE SIDE-PUMPED ND:YA1O3 LASER AT 1.08 AND 1.34 UM'
- IEEE TRANSACTION ON BIOMEDICAL ENGINEERING, vol. bme-28, no. 3, 1903 - 1981 pages 297-299, L.F.STOKES ET AL. 'Biomedical utility of 1.34 um Nd:YAG laser radiation'
- Notice technique donnant l' avis du docteur J.F. Dumon sur les performances du Lokki YAP laser.

## Description

### DOMAINE TECHNIQUE

La présente invention concerne le domaine technique des appareils conçus pour assurer l'émission pulsée d'un faisceau laser destiné à être utilisé dans le domaine médical au sens général.

Dans le domaine médical, le laser est utilisé principalement soit dans un mode d'incision ou de coupe analogue à celle d'un scalpel, soit dans un mode de coagulation ou de cautérisation des tissus humains.

### TECHNIOUE ANTERIEURE

Il est ainsi connu d'avoir recours à un laser Nd YAG dopé au Néodyme émettant à une longueur d'onde de 1,06 µm. Le rayonnement lumineux est absorbé par les tissus et permet d'obtenir un échauffement des tissus et l'effet cautérisant associé. A cette longueur d'onde, le faisceau laser est transmis par une fibre optique en silice qui permet son utilisation à l'intérieur du corps, sans nécessiter d'ouverture chirurgicale. Le fonctionnement impulsionnel autorise le perçage et l'abrasion des tissus durs. Toutefois, la coupe des tissus mous est lente et large, avec une zone affectée thermiquement trop profonde.

Il est connu, par ailleurs, d'utiliser un rayon laser à CO₂ qui permet d'obtenir la vaporisation des tissus, de sorte que le faisceau focalisé coupe dans la surface en faisant une incision. Un tel faisceau, qui présente une longueur d'onde de l'ordre de 10 µm, est fortement absorbé par les tissus. La coupe des tissus mous est bonne. Toutefois, les tissus durs sont parfois fissurés par la diffusion thermique du laser. De plus, le faisceau du laser à CO₂ ne se propage pas le long de fibres optiques flexibles, de sorte que l'amenée du faisceau doit se faire par l'intermédiaire d'une combinaison relativement complexe de tubes et de miroirs.

Afin d'associer les avantages des deux lasers ci-dessus, il a été proposé de réaliser un appareil intégrant les deux lasers du type ci-dessus. Toutefois, il doit être considéré que le laser à CO₂ conserve ses inconvénients limitant considérablement le domaine d'application d'un tel appareil.

La demande de brevet français FR 2 607 329 a proposé un laser Nd YAG équipé de moyens de sélection de la longueur d'onde du faisceau, à une valeur de 1,44 µm. A cette longueur d'onde, la lumière est mieux absorbée par l'eau contenue dans les tissus qu'à une longueur d'onde inférieure, par exemple égale à 1,32 µm ou 1,06 µm, qui sont les autres valeurs de transition d'un laser Nd YAG.

Lorsqu'un puissant faisceau laser à la longueur d'onde de 1,44 µm est focalisé sur des tissus, la surface des tissus s'échauffe et s'évapore de façon sensiblement identique à un faisceau laser à CO₂, réalisant ainsi une incision dans les tissus. L'inconvénient majeur de cet appareil laser réside dans son rendement faible nécessitant la mise en oeuvre de moyens d'excitation et de refroidissement surdimensionnés, afin d'obtenir une puissance laser suffisante pour la plupart des applications envisagées. Il en résulte un coût prohibitif limitant la diffusion d'un tel appareil laser.

L'examen par la Déposante des nombreuses solutions de l'état de la technique lui a permis de constater qu'il n'existait pas de lasers polyvalents dont le faisceau est transmissible par fibres optiques.

Aussi, la Déposante a eu le mérite de dresser la liste des caractéristiques que devait présenter un appareil laser à usage médical, afin de répondre aux besoins des utilisateurs. Il a été ainsi constaté que le faisceau laser devait posséder une longueur d'onde adaptée pour être absorbée aussi bien par les tissus durs que par les tissus mous, de manière à obtenir la coupe et la coagulation des tissus mous et l'ablation des tissus durs. Par tissus mous, il doit être compris, par exemple, les muqueuses, gencives, pulpes, parenchymes, muscles ou tumeurs, tandis que les tissus durs comprennent, par exemple, l'émail, la dentine, les calculs, les os, les cartilages ou les plaques d'athérome.

D'une manière supplémentaire, un faisceau laser à une telle longueur d'onde doit pouvoir se propager le long d'une fibre optique flexible, de manière à pouvoir être introduite à l'intérieur des organes creux et, plus généralement, de l'organisme humain ou animal. En outre, un tel appareil doit présenter un faible encombrement et son utilisation doit être aisée.

Pour de nombreuses applications, un appareil laser doit délivrer une puissance dans une gamme de 2 à 20 Watts. Pour délivrer cette puissance, un appareil laser doit posséder un bon rendement, afin d'éviter l'utilisation de moyens d'excitation et, par suite, de moyens de refroidissement, de puissance trop élevée, dont la mise en oeuvre augmente, de manière prohibitive, l'encombrement et le coût de fabrication d'un tel laser, le rendant inaccessible pour de nombreuses applications.

La Déposante a ainsi mis en évidence qu'il existait le besoin de disposer d'un appareil laser capable d'assurer la coupe des tissus mous et l'ablation des tissus durs, dont le faisceau est apte à être acheminé à l'aide d'une fibre optique flexible et dont le rendement est adapté pour permettre sa réalisation à un coût acceptable pour les nombreuses utilisations auxquelles l'appareil est destiné.

Après la formulation du besoin ci-dessus, la Déposante a mis au point un appareil laser répondant à la somme des caractéristiques techniques exigées dans les diverses applications envisagées.

### EXPOSE DE L'INVENTION

L'objet de l'invention concerne donc un appareil assurant l'émission pulsée d'un faisceau laser, du type comportant:
- un barreau laser monté dans une enveloppe réfléchissante,
- une lampe à décharge montée dans une enveloppe réfléchissante et assurant l'excitation pulsée du barreau laser, de manière que ce dernier délivre un rayonnement lumineux,
- un résonateur placé de part et d'autre du barreau et permettant rémission d'un rayonnement lumineux laser constitué d'impulsions de durée proche de la durée d'excitation du barreau, le résonateur comportant des moyens de sélection de la longueur d'onde du rayonnement lumineux laser émis.

Selon l'invention, l'appareil comprend :
- en tant que barreau laser, un cristal dopé au Néodyme d'Yttrium Aluminium Pérovskite de formule Nd : YAlO₃,
- et des moyens de sélection conçus pour favoriser l'émission du rayonnement lumineux à une longueur d'onde de 1,34 µm, de façon à assurer un effet thermique sur les tissus mous et les tissus durs.

Diverses autres caractéristiques ressortent de la description faite ci-dessous en référence aux dessins annexés qui montrent, à titre d'exemples non limitatifs, des formes de réalisation et de mise en oeuvre de l'objet de l'invention.

### BREVE DESCRIPTION DES DESSINS

La **fig. 1** est une vue schématique d'un exemple de réalisation d'un appareil laser selon l'invention.

La **fig. 2** est un diagramme montrant les caractéristiques de la puissance de différents lasers en fonction de la puissance de pompe associée.

### MEILLEURE MANIERE DE REALISER L'INVENTION

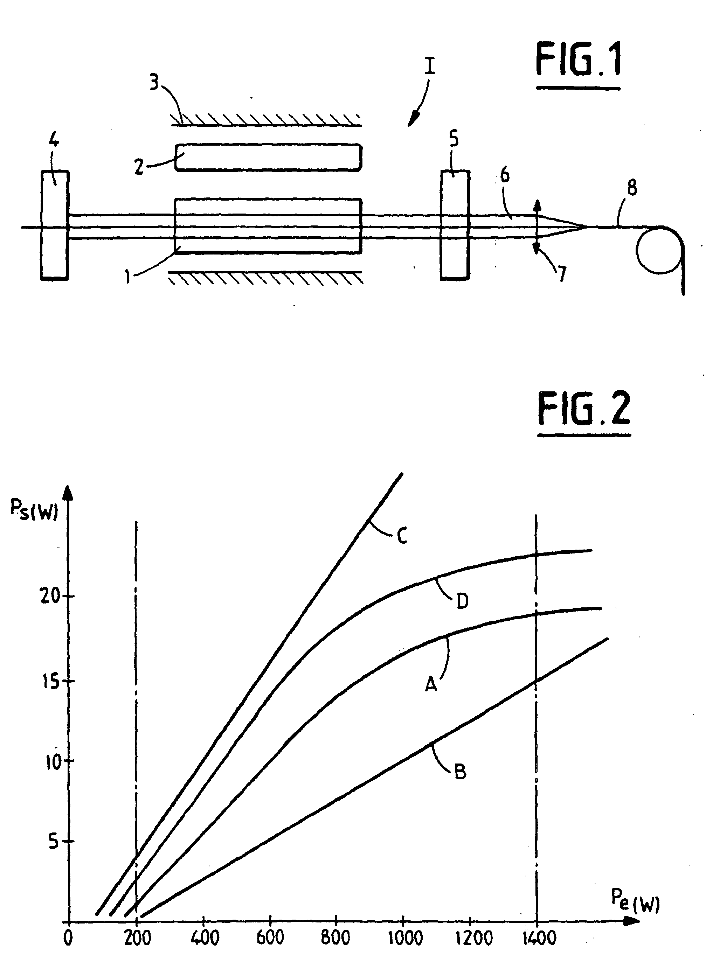

Tel que cela apparaît plus précisément à la **fig. 1,** le dispositif laser **I** selon l'invention comporte un milieu actif ou un barreau laser **1** qui est constitué avantageusement d'un cristal d'Yttrium Aluminium Pérovskite désigné par le sigle YAP, et appelé parfois également YALO₃ pour la dénomination Yttrium Aluminium Oxyde. Un tel cristal est dopé au Néodyme dans une gamme de pourcentage allant de 0,4 à 2 et, de préférence, sensiblement de l'ordre de 0,8 %. Un barreau laser YAP ou YALO₃ rayonne normalement à des valeurs de longueur d'onde égale à 1,07 ou 1,34 µm.

Le barreau laser **1** est associé à des moyens d'excitation comportant une lampe d'excitation **2** reliée à un circuit d'excitation pulsée de la lampe non représenté, et une enveloppe réfléchissante **3,** dans laquelle sont montés la lampe d'excitation **2** et le barreau **1.** La durée d'excitation, qui est réalisée par tous moyens appropriés connus en soi, est comprise entre 10 µs et 10 ms. Par exemple, la lampe d'excitation **2** est constituée par des diodes laser ou par une lampe à décharge au xénon ou au krypton. Avantageusement, la lampe est au xénon et son enveloppe est adaptée pour éliminer ou filtrer les rayonnements ultraviolets de la lampe de longueur d'onde inférieure à 350 nm. De préférence, l'enveloppe réfléchissante **3** filtre ou élimine les rayonnements ultraviolets de longueur d'onde inférieure à 350 nm. Bien entendu, il peut être envisagé de mettre en oeuvre tout autre moyen de filtration du rayonnement lumineux. Par exemple, un filtre peut être interposé entre la lampe **2** et le barreau **1**. La mise en oeuvre de tels moyens de filtration, seuls ou en combinaison, permet d'éviter l'apparition du phénomène de solarisation du cristal.

Le dispositif laser I comporte aussi un résonateur **4, 5** permettant l'émission d'un rayonnement lumineux laser **6**. Le résonateur est constitué de deux miroirs **4, 5** disposés de part et d'autre du barreau laser **1**. D'une manière avantageuse, le résonateur **4, 5** est conçu pour sélectionner la longueur d'onde du rayonnement lumineux émis par le barreau **1,** de manière à favoriser l'émission à la longueur d'onde de 1,34 µm, Les miroirs **4, 5** possèdent ainsi des coefficients de réflexion optimisés pour respectivement favoriser l'émission à 1,34 µm et empêcher l'émission à 1,07 µm. Par exemple, le miroir **4** est réfléchissant maximum à 1,34 µm, tandis que l'autre miroir **5** est réfléchissant entre 20 et 80 % à 1,34 µm et anti-reflet à 1,07 µm. De cette façon, le laser fonctionne en émission libre dite relaxée avec des impulsions de durée proche de la durée d'excitation et toute l'énergie laser est émise à la longueur d'onde de 1,34 µm.

Le faisceau laser **6** est focalisé par l'intermédiaire d'une lentille **7** sur l'entrée d'une fibre optique **8** qui le transmet jusqu'au tissu à traiter. Cette fibre optique possède un coeur en silice et, de préférence, en silice, dite sèche à faible teneur en ions OH.

L'appareil laser **I** selon l'invention présente ainsi la particularité de travailler à une longueur d'onde de 1,34 µm, pour laquelle les tissus absorbent bien le rayonnement lumineux. Il apparaît ainsi possible de couper des tissus mous avec des résultats comparables à ceux obtenus par un laser à CO₂ continu avec, toutefois, des avantages supplémentaires, tels que moins de fumée, meilleure hémostase et coagulation, et meilleure accessibilité due à la fibre optique. Il s'avère, également, qu'un tel appareil laser assure l'ablation des tissus durs avec un résultat équivalent à un laser Nd YAG impulsionnel. L'appareil laser **I** est donc à même d'exercer des effets thermiques sur les tissus biologiques, tels que coupe, coagulation et vaporisation des tissus mous ou perçage, abrasion et fusion des tissus durs.

Le laser selon l'invention présente un autre avantage qui est celui de son rendement relativement élevé par rapport aux autres lasers connus. Une illustration de cet avantage est apportée par la **fig. 2** qui montre les caractéristiques de la puissance du laser **Ps** en fonction de la puissance de pompe **Pe**, pour différents types de lasers placés dans des configurations semblables de pompage. Pour des valeurs de puissance de pompe comprises sensiblement entre 200 et 1 400 Watts, la puissance laser (courbe A) obtenue pour l'appareil laser selon l'invention à la longueur d'onde de l'ordre de 1,34 µm est supérieure à celle (courbe B) d'un laser Nd YAG à 1,32 µm. En pratique, il est prévu d'obtenir un rendement supérieur à 1 % et, de préférence, de l'ordre de 2 %, de telles valeurs ne pouvant pas être atteintes par un laser YAG à 1,32 µm (courbe B). Même si les rendements obtenus pour un laser Nd YAG à 1,06 µm (courbe C) ou pour un laser Nd YAP à 1,07 µm (courbe D) sont supérieurs à ceux du laser selon l'invention fonctionnant à 1,34 µm, il doit être considéré que ces deux longueurs d'ondes (1,06 et 1,07) se trouvent insuffisamment absorbées par les tissus pour assurer les effets thermiques souhaités. Le dispositif I selon l'invention présente ainsi un bon rendement, pour des puissances laser comprises entre 2 et 20 Watts, et un rayonnement lumineux dont la longueur d'onde s'avère, par ailleurs, bien absorbée pour assurer des fonctions de cautérisation et de coupe des tissus.

### POSSIBILITE D'APPLICATION INDUSTRIELLE

L'objet de l'invention trouve une application particulièrement avantageuse dans le domaine dentaire, afin d'assurer notamment des opérations de chirurgie, des traitements de fonds de cavités ou des traitements de canaux. L'appareil laser selon l'invention trouve d'autres applications dans le domaine médical, notamment, en orthopédie (nucléotomie percutanée, méniscectomie), en neurochirurgie, en gynécologie (chirurgie par voie coelioscopique) ou en O.R.L (larynx, sinus ou oreille interne).

## Revendications

1. Appareil assurant l'émission puisée d'un faisceau laser (**6**), du type comportant :
- un barreau laser (**1**) monté dans une enveloppe réfléchissante (**3**),
- une lampe à décharge **(2)** montée dans une enveloppe réfléchissante et assurant l'excitation pulsée du barreau laser (**1**), de manière que ce dernier délivre un rayonnement lumineux,
- un résonateur (**4,5**) placé de part et d'autre du barreau (**1**) et permettant l'émission d'un rayonnement lumineux laser constitué d'impulsions de durée proche de la durée d'excitation du barreau, le résonateur comportant des moyens de sélection de la longueur d'onde du rayonnement lumineux laser émis,
**caractérisé en ce qu'**il comprend :
• en tant que barreau laser (**1**), un cristal dopé au Néodyme d'Yttrium Aluminium Pérovskite de formule Nd : YAlO₃,
• et des moyens de sélection conçus pour favoriser l'émission du rayonnement lumineux à une longueur d'onde de 1,34 µm, de façon à assurer un effet thermique sur les tissus mous et les tissus durs.

2. Appareil selon la revendication 1, **caractérisé en ce que** les moyens de sélection de la longueur d'onde sont constitués par deux miroirs (**4, 5**) constitutifs du résonateur et présentant des coefficients de réflexion respectivement optimisés pour favoriser une émission du rayonnement lumineux à une longueur d'onde de 1,34 µm et pour empêcher l'émission du rayonnement lumineux à une longueur d'onde de 1,07 µm.

3. Appareil selon la revendication 2, **caractérisé en ce que** l'un des miroirs (**4**) est réfléchissant maximum à la longueur d'onde de 1,34 µm, tandis que l'autre des miroirs (**5**) est partiellement réfléchissant à la longueur d'onde de 1,34 µm et anti-reflet à la longueur d'onde de 1,07 µm.

4. Appareil selon la revendication 1 ou 3, **caractérisé en ce que** les moyens d'excitation (**2, 3**) comportent des moyens de filtration du rayonnement lumineux de longueur d'onde inférieure à 350 nm.

5. Appareil selon la revendication 4, **caractérisé en ce que** les moyens de filtration du rayonnement lumineux sont assurés par une enveloppe de la lampe à décharge (**2**).

6. Appareil selon la revendication 4, **caractérisé en ce que** les moyens de filtration du rayonnement lumineux sont assurés par l'enveloppe réfléchissante (**3**).

7. Appareil selon la revendication 4, **caractérisé en ce que** les moyens de filtration du rayonnement lumineux sont assurés par un filtre interposé entre la lampe (**2**) et le barreau (**1**).

8. Appareil selon la revendication 1, **caractérisé en ce que** les moyens de sélection sont conçus pour favoriser l'émission du rayonnement lumineux à une longueur d'onde de 1,34 µm, de façon à assurer la coupe et la coagulation des tissus mous et l'ablation des tissus durs.

9. Appareil selon la revendication 1, **caractérisé en ce que** le barreau (**1**) émet un rayonnement lumineux laser constitué d'impulsions de durée proche de la durée d'excitation du barreau, comprise entre 10 µs et 10 ms.

## Claims

1. A device providing pulsed emission of a laser beam (6), the device being of the type comprising:
- a laser rod (1) mounted in a reflecting envelope (3);
- a discharge lamp (2) mounted in a reflecting envelope (3) and ensuring the pulsed excitation of the laser rod (1) to cause it to deliver light radiation;
- a resonator (4, 5) placed on either side of the rod (1) and enabling laser light radiation to be emitted which consists of pulses of duration close to the excitation duration of the rod, the resonator including selection means for selecting the wavelength of the emitted laser light radiation;
the device being **characterised in that** it comprises:
• as the laser rod (1), a neodymium-doped crystal of yttrium aluminium perovskite of formula Nd: YAlO₃; and
• selection means designed to favour the emission of the light radiation at a wavelength of 1.34 µm, so as to ensure a thermal effect both on soft tissue and on hard tissue.

2. A device according to claim 1, **characterised in that** the wavelength selection means are constituted by two mirrors (4, 5) constituting the resonator and having reflection coefficients respectively optimised to favour emission of light radiation at a wavelength of 1.34 µm and to prevent light radiation being emitted at a wavelength of 1.07 µm.

3. A device according to claim 2, **characterised in that** one of the mirrors (4) has a reflection maximum at a wavelength of 1.34 µm, while the other mirror (5) is partially reflective at the wavelength of 1.34 µm, and antireflective at the wavelength of 1.07 µm.

4. A device according to claim 1 or 3, **characterised in that** the excitation means (2, 3) include means for filtering light radiation at a wavelength shorter than 350 nm.

5. A device according to claim 4, **characterised in that** the means for filtering light radiation are provided by the envelope of the discharge lamp (2).

6. A device according to claim 4, **characterised in that** the means for filtering light radiation are provided by the reflecting envelope (3).

7. A device according to claim 4, **characterised in that** the means for filtering light radiation are provided by a filter interposed between the lamp (2) and the rod (1).

8. A device according to claim 1, **characterised in that** the selection means are designed to favour the emission of the light radiation at a wavelength of 1.34 µm, so as to ensure the cutting and coagulation of the soft tissue and the removal of the hard tissue.

9. A device according to claim 1, **characterised in that** the rod (1) emits a laser light radiation which consists of pulses of duration close to the excitation duration of the rod, as comprised between 10 µs and 10 ms.

## Patentansprüche

1. Vorrichtung zur gepulsten Emission eines Laserstrahls (6) mit:
- einem in einer reflektierenden Umhüllung (3) montierten Laserstab (1),
- einer in einer reflektierenden Umhüllung montierten Entladungslampe (2) zur gepulsten Anregung des Laserstabs (1), so dass durch diesen eine Lichtstrahlung abgegeben wird,
- einem Resonator (4, 5), der zu beiden Seiten des Stabs (1) platziert ist und die Emission einer Laserstrahlung ermöglicht, die aus Impulsen gebildet ist, deren Dauer der Dauer der Anregung des Stabs ähnlich ist, wobei der Resonator Mittel zur Auswahl der Wellenlänge der emittierten Laserstrahlung umfasst,
**dadurch gekennzeichnet, dass** sie umfasst:
• als Laserstab (1) ein mit Neodym-Yttrium-Aluminium-Perowskit dotiertes Kristall mit der Formel Nd:YalO₃,
• und Auswahlmittel, die so konzipiert sind, dass die Emission der Lichtstrahlung mit einer Wellenlänge von 1,34 µm begünstigt wird, um in weichen und festen Geweben eine Wärmewirkung zu erreichen.

2. Vorrichtung nach Anspruch 1,
**dadurch gekennzeichnet, dass** die Mittel zur Auswahl der Wellenlänge von zwei den Resonator bildenden Spiegeln (4, 5) gebildet sind und jeweils optimierte Reflexionskoeffizienten aufweisen, um eine Emission der Lichtstrahlung mit einer Wellenlänge von 1,34 µm zu begünstigen und die Emission der Lichtstrahlung mit einer Wellenlänge von 1,07 µm zu verhindern.

3. Vorrichtung nach Anspruch 2,
**dadurch gekennzeichnet, dass** einer der Spiegel (4) maximal bei der Wellenlänge von 1,34 µm reflektierend ist, wogegen der andere Spiegel (5) bei der Wellenlänge von 1,34 µm teilweise reflektierend und bei der Wellenlänge von 1,07 µm nicht reflektierend ist.

4. Vorrichtung nach Anspruch 1 oder 3,
**dadurch gekennzeichnet, dass** die Anregungsmittel (2, 3) Mittel zum Filtern der Lichtstrahlung mit einer Wellenlänge von unter 350 nm umfassen.

5. Vorrichtung nach Anspruch 4,
**dadurch gekennzeichnet, dass** die Mittel zum Filtern der Lichtstrahlung durch eine Umhüllung der Entladungslampe (2) gewährleistet sind.

6. Vorrichtung nach Anspruch 4,
**dadurch gekennzeichnet, dass** die Mittel zum Filtern der Lichtstrahlung durch die reflektierende Umhüllung (3) gewährleistet sind.

7. Vorrichtung nach Anspruch 4,
**dadurch gekennzeichnet, dass** die Mittel zum Filtem der Lichtstrahlung durch einen zwischen der Lampe (2) und dem Stab (3) eingefügten Filter gewährleistet sind.

8. Vorrichtung nach Anspruch 1,
**dadurch gekennzeichnet, dass** die Auswahlmittel so konzipiert sind, dass die Emission der Lichtstrahlung mit einer Wellenlänge von 1,34 µm begünstigt wird, um das Schneiden und Koagulieren von weichen Geweben und die Ablation von festen Geweben zu gewährleisten.

9. Vorrichtung nach Anspruch 1,
**dadurch gekennzeichnet, dass** der Stab (1) eine Laserstrahlung emittiert, die aus Impulsen gebildet ist, deren Dauer der Dauer der Anregung des Stabs ähnlich ist, die zwischen 10 µs und 10 ms liegt.
